# EUROPEAN PATENT SPECIFICATION

(11) **EP 3 487 508 B1**
(45) Date of publication and mention of the grant of the patent: **13.09.2023**
(21) Application number: 17743314.1
(22) Date of filing: 20.07.2017
(51) Int. Cl.: A61K 31/724, C08B 37/16, A61P 31/12, A61P 31/14, A61P 31/18, A61P 31/20, A61P 31/22

(54) **VIRUCIDAL COMPOUNDS AND USES THEREOF**
VIRUZIDVERBINDUNGEN UND VERWENDUNGEN DAVON
COMPOSÉS VIRUCIDES ET LEURS UTILISATIONS

(30) Priority: 22.07.2016 EP 16180726
(43) Date of publication of application: 29.05.2019
(73) Proprietor: Ecole Polytechnique Fédérale de Lausanne (EPFL), 1015 Lausanne (CH)
(72) Inventor: STELLACCI, Francesco, 1110 Morges (CH); JONES, Samuel, SK7 6ER Stockport (CH)
(74) Representative: Vossius & Partner Patentanwälte Rechtsanwälte mbB
(86) International application number: PCT/EP2017/068291
(87) International publication number: WO 2018/015465

(56) References cited:
- WO-A1-2015/179963
- WO-A2-2005/042584
- US-A1- 2016 326 270
- SERGEY SHITYAKOV ET AL: "Sevoflurane-Sulfobutylether-[beta]-Cyclod extrin Complex: Preparation, Characterization, Cellular Toxicity, Molecular Modeling and Blood-Brain Barrier Transport Studies", MOLECULES, vol. 20, no. 6, 3 June 2015 (2015-06-03), pages 10264-10279, XP055361090, DOI: 10.3390/molecules200610264
- KIRSCHNER D ET AL: "Fine tuning of sulfoalkylated cyclodextrin structures to improve their mass-transfer properties in an aqueous biphasic hydroformylation reaction", JOURNAL OF MOLECULAR CATALYSIS A: CHEMICAL, vol. 286, no. 1-2, 1 May 2008 (2008-05-01) , pages 11-20, XP022649936, ELSEVIER, AMSTERDAM, NL ISSN: 1381-1169, DOI: 10.1016/J.MOLCATA.2008.01.038 [retrieved on 2008-02-07]
- DEBOUZY, J.-C. ET AL: "Substituted cyclodextrins as chelating reagents for yperite, ethers and thioethers", ANNALES PHARMACEUTIQUES FRANCAISES, vol. 58, no. 1, 2000, pages 20-23, XP009500352, ISSN: 0003-4509
- DATABASE MEDLINE [Online] US NATIONAL LIBRARY OF MEDICINE (NLM), BETHESDA, MD, US; October 1997 (1997-10), WITVROUW M ET AL: "Sulfated polysaccharides extracted from sea algae as potential antiviral drugs.", XP002774102, Database accession no. NLM9352294 & WITVROUW M ET AL: "Sulfated polysaccharides extracted from sea algae as potential antiviral drugs.", GENERAL PHARMACOLOGY OCT 1997, vol. 29, no. 4, October 1997 (1997-10), pages 497-511, ISSN: 0306-3623
- PAUWELS R ET AL: "Development of vaginal microbicides for the prevention of heterosexual transmission of HIV.", JOURNAL OF ACQUIRED IMMUNE DEFICIENCY SYNDROMES AND HUMAN RETROVIROLOGY , 1 March 1996 (1996-03-01), pages 1-13, XP002774103, Retrieved from the Internet: URL:http://journals.lww.com/jaids/Fulltext /1996/03010/Development_of_Vaginal_Microbi cides_for_the.1.aspx [retrieved on 2017-09-26]
- CHIARA URBINATI ET AL: "Polyanionic Drugs and Viral Oncogenesis: a Novel Approach to Control Infection, Tumor-associated Inflammation and Angiogenesis", MOLECULES, vol. 13, no. 11, 6 November 2008 (2008-11-06), pages 2758-2785, XP055409943, DOI: 10.3390/molecules13112758
- DATABASE MEDLINE [Online] US NATIONAL LIBRARY OF MEDICINE (NLM), BETHESDA, MD, US; April 1990 (1990-04), DE CLERCQ E: "Selective virus inhibitors.", XP002774104, Database accession no. NLM1693749 & DE CLERCQ E: "Selective virus inhibitors.", MICROBIOLOGICA APR 1990, vol. 13, no. 2, April 1990 (1990-04), pages 165-178, ISSN: 0391-5352
- Y. NAGASE ET AL: "Protective Effect of Sulfobutyl Ether [beta]-Cyclodextrin on DY-9760e-Induced Hemolysis In Vitro", JOURNAL OF PHARMACEUTICAL SCIENCES, vol. 91, no. 11, 1 November 2002 (2002-11-01), pages 2382-2389, XP055409295, ISSN: 0022-3549, DOI: 10.1002/jps.10236

## Description

### Field of the Invention

The invention relates to virucidal compounds, virucidal compositions comprising thereof and uses thereof in treatment of viral infections, for sterilizations and for disinfections.

### Background of the Invention

Viruses are the most abundant biological entities on Earth and are capable of infecting all types of cellular life including animals, plants, bacteria and fungi. Viral infections kill millions of people every year and contribute substantially to health care costs. The negative impact viruses can have on society is significant. From viral infections of food, crops, infection of livestock, to the serious health impacts viral infections, such as HIV, Ebola or Zika, have on humans. Some viral infections have also been linked with cancer, such as the human papillomavirus (HPV), which is linked with cervical cancer: the fourth most common cancer in women.

The best way to fight viral infection is vaccination. However, vaccines are not always available and in underdeveloped countries having sufficient vaccine coverage can be a significant challenge. Additionally, once infected, vaccination is no longer useful and drugs are needed to help the immune system to fight the infection.

Anti-viral drugs, which act by disrupting the intracellular pathways used by viruses to replicate, are often prescribed to aid the immune systems fight against the infection. Current antiviral therapeutics, which can be found in the form of small molecules, proteins able to stimulate the immune response (e.g. interferon), or oligonucleotides (REF-virucidal paper 5). These therapeutics focus on only a few viruses, such as, Human Immunodeficiency Virus (HIV), Hepatitis B Virus (HBV), Hepatitis C Virus (HCV), Hu- man Cytomegalovirus (HCMV), Herpes Simplex Virus (HSV), Varicella Zoster Virus (VZV), and influenza virus. Specific antiviral treatments are lacking for the majority of viruses. Current therapeutic approaches typically act intracellularly on viral enzymes, that are es sential for viral replication, but differ from the hosts enzymes allowing for a degree of selectivity. However, since viruses largely depend on the biosynthetic machinery of the infected cells for their replication, the specificity of antiviral drugs is often far from ideal resulting in a general intrinsic toxicity associated with such treatment. Additionally, viruses mutate rapidly and on account of error prone replication machinery, they often develop resistance to such antivirals. The use of virus specific proteins as a target of antiviral drugs makes it difficult to develop broad-spectrum antivirals acting on a large number of viruses that are phylogenetically unrelated and structurally different (e.g. viruses with or without an outer lipid envelope). It can be reasoned that the ideal viral drug is a broad-spectrum non-toxic material that acts outside the host and irreversibly inhibits viruses, i.e. a virucidal drug. Drugs that are active outside of the cell environment currently only display virustatic properties, in that inhibition is achieved via a reversible binding to a host cells attachment receptors or the virus ligands, leading to reduction in cell-virus interactions. The reversible nature of the interaction makes such materials inadequate for medical applications, as upon dilution the drug is released from the cell or virus, and no permanent inactivation occurs, allowing the virus to once again infect. Heparan sulphate (HS), a sulfonated linear polysaccharide, has been shown to play an important role as the cell surface attachment receptor for a large number of viruses, including but not limited to; human immunodeficiency virus (HIV), herpes simplex virus (HSV) 1 and 2, HPV, dengue virus (DENV), adenovirus, and hepatitis-C.5 A number of attempts have been made to synthetically produce HS-like or HS binding materials that have potential to act as anti-virals. Only three polyanionic anti-HIV-1 microbicides, that are all highly sulfonated polymers like HS, have reached phase III clinical trials (i.e. polysulfonated PR02000, the polysulfated Carraguard, and cellulose Ushercell). None were able to prevent vaginal HIV-1 transmission and even increased the rate of infection in some cases. One possible explanations is that their effect was simply virustatic and hence vaginal and seminal fluids lead to the dilution of both the viruses and the active compounds, which resulted in the complete loss of binding and active virus release.

Another approach for the treatment of viruses exists but is currently not medically applicable. It is a broad-spectrum approach that is not affected by viral mutations and is effective againstall known viruses. It is referred to as virucidal action and results in a virus being rendered inert upon contact with the virucidal material. It should be noted that virucidal drugs have irreversible effects on the virus; indeed their effect is retained even if dilution occurs after the initial interaction with the virus. There is a vast literature on many virucidal materials ranging from simple detergents, to strong acids, or more refined polymers, and nanoparticles in some cases capable of releasing ions are known virucidal molecules. For example, virucidal properties are common in sterilising solutions, for the cleaning of medical equipment or other surfaces that may have come in contact with viruses. In all of these cases, the approaches used to irreversibly inhibit the virus have intrinsic cellular toxicity side effects. The challenge is to find virucidal materials/molecules that have minimal side effects on the host, and are thus able to act as virucidal drugs ideally in a broad-spectrum manner. Currently, there is no approved drug that shows virucidal activity.

The present invention was able to solve this problem by providing virucidal compounds having unique properties.

Document WO 2015/179963 discloses certain cyclodextrin-based polyanionic and non-ionic dendrimers.

Shityakov and coworkers disclose certain Sevoflurane-Sulfobutylether-[beta]-cyclodextrin complex (Molecules, vol. 20, no. 6, 3 June 2015, pages 10264-10276).

Kirschner and coworkers disclose fine-tuning of certain sulfoxylated cyclodextrin structures to improve their mass-transfer properties in an aqueous biphasic hydroformylation reaction (Journal of Molecular Catalysis A: Chemical, vol. 286, no 1-2, May 2008, pages 11-20).

Debouzy and coworkers disclose certain substituted cyclodextrins as chelating reagents for yperite, ethers and thioethers (Annales Pharmaceutiques Francaises, vol 58, no. 1, 2000, pages 20-23).

Witvrouw et a; disclose certain sulfated polysaccharides extracted from sea algae as potential antiviral drugs (General Pharmacology, October 1997, vol 29, no. 4, pages 497-511).

Pauwels et al disclose development of certain vaginal microbicides for the prevention of heterosexual transmission of HIV (Journal of Acquired Immune Deficiencyu Syndromes and Human Retrovirology, 1 March 1996, pages 1 to 13).

Chiara Urbinati et al disclose a novel approach to control infection, tumor-associated inflammation and angiogenesis with polyanionic drugs (Molecules, vol. 13, no. 11, 6 November 2008, pages 2758-2785).

Nagase et al. disclose protective effect of certain sulfobutyl ether [beta]-cyclodextrin on DY-9760e-induced Hemolysis in vitro (journal of Pharmaceutical Sciences, vol. 91, no. 11, 1 November 2002, pages 2382-2389).

Document US 2016/326270 discloses a method for preparing certain sulfobutyl ether-[beta]-cyclodextrin.

Document WO 2005/042584 discloses certain sulfoalkyl ether-alkyl ether cyclodextrin derivatives.

### Summary of the Invention

The invention is as characterized in the appended set of claims.

### Brief description of figures

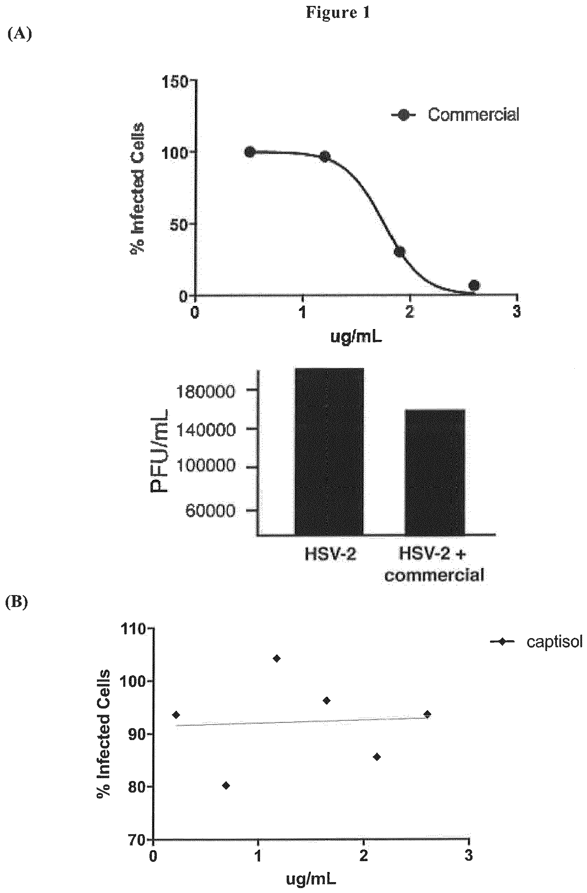

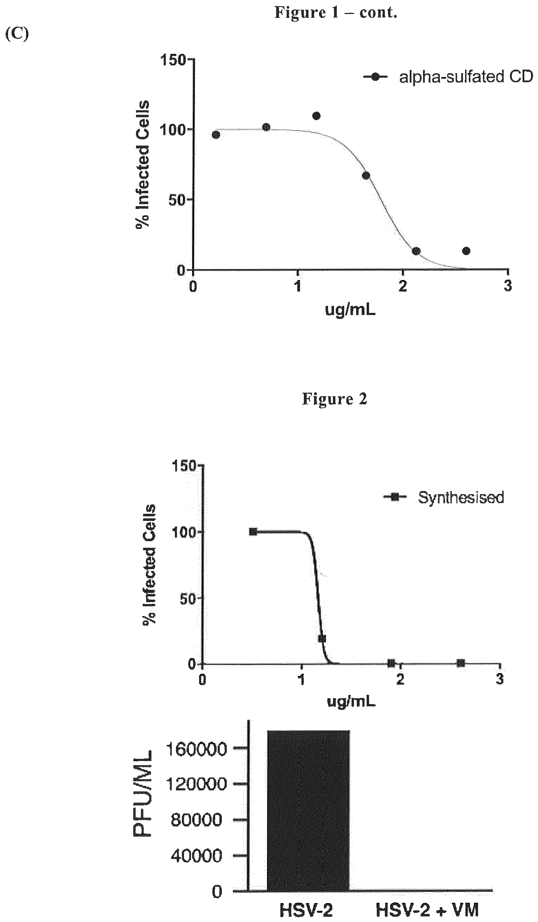

Figure 1 (A) shows beta-cyclodextrin sulfonate (Sigma Aldrich) tested against HSV-2 in (top) a dose response assay and (bottom) a virucidal assay. Inhibition is observed at high concnetrations but no virucidal effect is observed. (B) shows a compound disclosed in the patent application US2005209189A1. No interaction with viruses up to 400ug/mL. (C) alpha-sulfated cyclodextrin (Sigma Aldrich)
Figure 2 (A) shows synthesised CD derivative (**CD2** of the invention) tested against HSV-2. Top - dose response and bottom - virucidal assay. Virucidal effect observed.

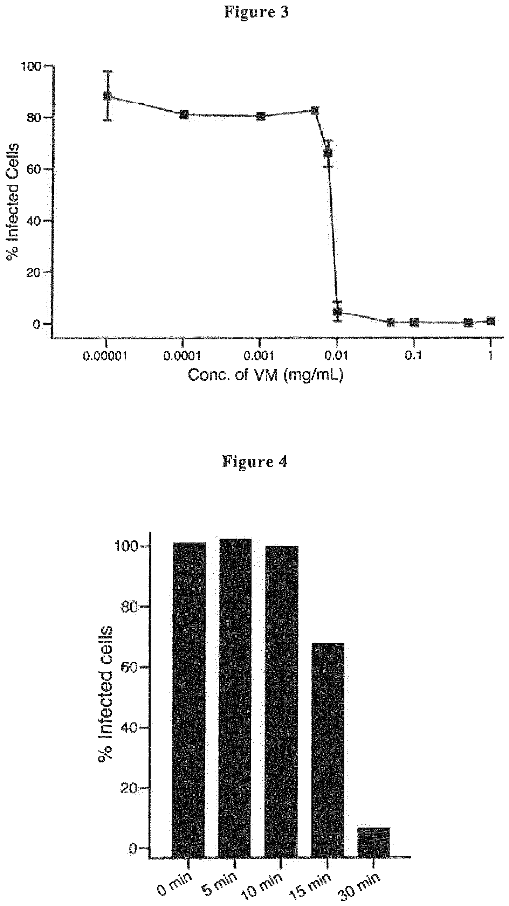

**Figure 3** shows dose response curve for CD2 of the invention against Lenti Virus.
**Figure 4** shows virucidal time study showing almost complete virucidal effect after ~ 30 min.

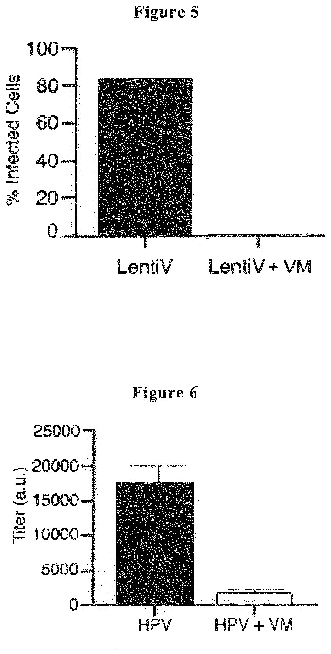

**Figure 5** shows virucidal effect of CD2 of the invention against Lenti virus.
**Figure 6** shows virucidal effect of CD2 of the invention against human papillomavirus (HPV).

### Detailed description of the Invention

Che publications and applications discussed herein are provided solely for their disclosure prior to the filing date of the present application. Nothing herein is to be construed as an admission that the present invention is
not entitled to antedate such publication by virtue of prior invention. In addition, the materials, methods, and examples are illustrative only and are not intended to be limiting.

In the case of conflict, the present specification, including definitions, will control.

Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise" is generally used in the sense of include, that is to say permitting the presence of one or more features or components. In addition, as used in the specification and claims, the language "comprising" can include analogous embodiments described in terms of "consisting of " and/or "consisting essentially of'.

As used in the specification and claims, the term "and/or" used in a phrase such as "A and/or B" herein is intended to include "A and B", "A or B", "A", and "B".

As used in the specification and claims, the singular form "a", "an" and "the" include plural references unless the context clearly dictates otherwise.

As used herein, the term "virustatic" refers to inhibition of the growth and/or development and/or the replication of viruses, which is different from destruction of viruses. Typically, the inhibition effect is obtained by coating of virus capsids or blocking cell surface receptors effectively, thereby creating a barrier to interaction between a virus and a cell. However, a virus remains active, can be released and can further infect cells.

As used herein, the term "virucidal" refers to neutralization and/or destructions of a virus. Interaction with virucidal compounds alters the virus, rendering it inert, and thereby prevents further infections.

As used herein, the term "biocompatible" refers to compatible with living cells, tissues, organs, or systems, and having no risk of injury, toxicity, or rejection by the immune system.

The term "alkyl" used alone or in combination with other groups should be understood to include straight chain and branched hydrocarbon groups having from 4 to 50, preferably 6 to 20 carbon atoms. Alkyl groups may be optionally substituted with one or more substituents. Non-limiting examples of suitable alkyl groups include methyl, ethyl, n-propyl, i-propyl, n-butyl, i-butyl, t-butyl, n-pentyl, n-hexyl.

A viral capsid is formed, via self-assembly, inside infected cells and is held in metastable state i.e. a long-lived but not eternal. When a virus attaches to a host cell a cascade of events result in the capsid unfolding and the genetic material being released into the cell. A metastable state is therefore necessary, as the capsid must easily unfold when a suitable host cell has been identified. Each binding event is controlled by supramolecular interactions, with the strength, direction and order of binding events being important for effective viral infectivity. It has been shown that the first attachment in the cascade occurs between viral ligands and receptors found on the surface of host cells, such as Heparan Sulfate (HS) cellular receptor for a large number of heparan sulfate proteoglycan (HSPG) binding viruses. Whilst viruses are able to mutate rapidly the cell attachment receptor is highly conserved both in and trough viruses.

A biomimetic strategy has been developed to develop broad-spectrum virucidal compounds. To limit toxicity, known bio-toxic approaches have been avoided and instead it has been concentrated on mimicking cell-receptor, so as to strongly attach to their corresponding viral ligand and generate localviral deformation that would ultimately lead to irreversible viral mutations, possibly leading to viral disassembly. To achieve broad-spectrum efficacy, it has been focused on virus-cell interactions that are common to many viruses. One of these interactions is that between viruses and cell-surface attachment receptors that represent the very first step of the virus replicative cycle. Many viruses, including HIV-1, HSV, HCMV, HPV, Respiratory syncytial virus (RSV) and filoviruses (virucidal refs 21), exploit heparan sulfate proteoglycans (HSPGs) as attachment receptors, as HSPG sare expressed on the surface of almost all eukaryotic cell types. The binding takes place between viruses and HSPGs usually occurs via binding of stretches of basic amino acids on viral proteins (basic domains) and the negatively charged sulfated groups of heparan sulfate (HS) chains in the glycocalix of the cell surface.

By designing a compound with a very high density of long sulfonic acid terminated molecules, in order to induce strong multivalent binding to the virus and in order to imitate the cell attachment receptor, such as Heparan Sulfate (HS) receptor, it was surprisingly found that such compound upon interaction with a virus leads to an irreversible change in the virus leading to the virus destruction and the permanent loss of viral infectivity. For example, it was unexpectedly found that by a chemical modification of a cyclic sugar unit, such as cyclodextrin, it is possible to provide a biocompatible virucidal molecule that shows virucidal properties at low concentrations against a wide range of viruses including herpes simplex virus (HSV), human papillomavirus virus (HPV), respiratory syncytial virus (RSV), dengue virus and lentivirus (a human immunodeficiency virus (HIV) derived virus).

An aspect of the invention provides a virucidal compound consisting of multiple (several) alkyl sulfate groups that provide the attachement receptor for HSPG binding viruses. In some embodiments, the virucidal compound consists of a very high density of alkyl sulfate groups. In some embodiments, the virucidal compound consists of several (at least six) alkyl sulfate groups in close proximity.

In a preferred embodiment, alkyl sulfate group is -Z-CH₂-(CH₂)_{y}-SO₃⁻, wherein Z is O or S and y is at least 4, preferably y is 4 to 20, preferably y is 7 to 11, most preferably y is 10. In other embodiments, y is at least 6, at least 7, at least 8, at least 9, at least 10, at least 11. In other embodiments, y is at maximum 100, at maximum 70, at maximum 50, at maximum 25, at maximum 20, at maximum 15.

In other embodiments, the virucidal compounds of the invention have a molecular weight of about 3 kDa.

The term "a virucidal compound consisting of multiple (several) alkyl sulfate groups" means that some of the OH groups on the compound are converted to OR groups, wherein R is - Z-CH₂-(CH₂)_{y}-SO₃⁻, wherein Z is O or S and y is at least 4, preferably y is 4 to 20, preferably y is 7 to 11, most preferably y is 10. In other embodiments, y is at least 6, at least 7, at least 8, at least 9, at least 10, at least 11. In other embodiments, y is at maximum 100, at maximum 70, at maximum 50, at maximum 25, at maximum 20, at maximum 15.

Contrary to the general knowledge, it was unexpectedly found that the specific lengh of the alkyl sulfate groups, i.e. 4 to 20 carbons, preferably 7 to 11, provide not only attachement receptor for HSPG binding viruses, but also provide virucidal effect, which is different from the known virustatic effect.

In some embodiments, the virucidal compound is a virucidal polymeric biocompatible compound.

The polymer in the polymeric biocompatible compounds of the invention can be both synthetic and natural polymers. In an embodiment of the invention, the synthetic polymers are selected from the group consisting of poly(ethylene glycol) (PEG), poly(vinyl alcohol) (PVA), poly(acrylamide) (PAAm), poly(n-butyl acrylate), poly-(**α**-esters), (PEG-b-PPO-b-PEG), poly(N-isopropylacrylamide) (pNIPAAM) and polylacticglycolic acid (PLGA). In another embodiment of the invention, the natural polymers are selected from the group consisting of dextran, dextrins, glucose, cellulose and cyclodextrins. In a preferred embodiment, the natural polymer is alpha-cyclodextrin, beta-cyclodextrin and gamma-cyclodextrin.

The polymer in the polymeric biocompatible compounds of the invention can be also a dendrimer (hyperbranched polymer). The polymeric biocompatible compounds can be micelles, particles, or hydrogels.

Cyclodextrins (CDs) are naturally occurring cyclic glucose derivatives consisting of alpha(14)-linked glucopyranoside units. Their cyclic structure creates a truncated cone shape with theprimary hydroxyls of the glucose units on the narrow face and the secondary hydroxyls on the wider face. Each face can be readily and independently functionalised as the secondary hydroxyl groups have a strong hydrophilic character, which influences their reactivity. The most commonly used natural CDs have 6-, 7-, and 8-glucopyranoside units, referred to as alpha, beta and gamma respectively. Because of the cyclic structure of CDs, they have a hydrophobic cavity capable of forming supramolecular inclusion complexes with guest molecules. As CDs are naturally occurring, readily functionalised, have a cavity for guest inclusion and are biocompatible, they have found use in many commercial applications including drug delivery, air fresheners, etc.

The difference in reactivity of each face of CDs has been used for the synthesis of a wide range of modified cyclodextrins. The primary face of CDs is more readily modified, with control over the degree and location of substitution being possible. CD derivatives that bear a good leaving group, such as halogenated CDs, are important intermediates in CD functionalisation. By replacing all of the primary hydroxyl units of CDs with iodo-units gives an intermediate that allows for complete functionalisation of the primary face, whilst leaving the secondary hydroxyls and the rigid truncated cone shape in tact. In one embodiment, heptakis-6-iodo-6-deoxy-beta-cyclodextrin was synthetized followed by reaction with mercaptoundecaosulphonate (MUS) to yield a CD functionalised on the primary face with undecanaosulfonate groups. It is then possible to independently modify the secondary face of the cyclodextrin to introduce further solubilising groups, dye molecules, polymers, etc.

Another aspect of the invention discloses virucidal compound of formula (I) 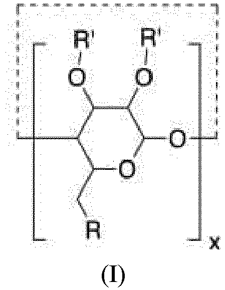 wherein
x is 6, 7 or 8
R is -Z-CH₂-(CH₂)_{y}-SO₃⁻
Z is O or S
y is at least 4, preferably y is 4 to 20, preferably y is 7 to 11, most preferably y is 10. In other embodiments, y is at least 6, at least 7, at least 8, at least 9, at least 10, at least 11. In other embodiments, y is at maximum 100, at maximum 70, at maximum 50, at maximum 25, at maximum 20, at maximum 15.
R' is selected from the group consisting of H, -(CH₂)y-SO₃⁻, -(CH₂)-COOH, polymer, such as PEG or other water solubilising group. Preferably R' is H.

It is understood that the virucidal compound of formula (I) is a cyclic compound, for example cyclodextrin.

The virucidal compounds of the invention provide virucidal activity at low concentrations, such as at micro molar levels and/or nano molar levels, against a wide range of viruses, such as herpes simplex virus (HSV), human papillomavirus virus (HPV), respiratory syncytial virus (RSV), dengue virus and lentivirus (a human immunodeficiency virus (HIV) derived virus).

Another aspect of the invention discloses a pharmaceutical composition comprising an effective amount of one or more virucidal compounds of the invention and at least one pharmaceutically acceptable excipient, carrier and/or diluent.

As to the appropriate excipients, carriers and diluents, reference may be made to the standard literature describing these, e.g. to chapter 25.2 of Vol. 5 of "Comprehensive Medicinal Chemistry", Pergamon Press 1990, and to "Lexikon der Hilfsstoffe für Pharmazie, Kosmetik und angrenzende Gebiete", by H.P. Fiedler, Editio Cantor, 2002. The term "pharmaceutically acceptable carrier, excipient and/or diluent" means a carrier, excipient or diluent that is useful in preparing a pharmaceutical composition that is generally safe, and possesses acceptable toxicities. Acceptable carriers, excipients or diluents include those that are acceptable for veterinary use as well as human pharmaceutical use. A "pharmaceutically acceptable carrier, excipient and/or diluent" as used in the specification and claims includes both one and more than one such carrier, excipient and/or diluent.

Optionally, the pharmaceutical composition of the present invention further comprises one or more additional active agents, preferably anti-viral agents.

The compounds of the invention that are used in the methods of the present invention can be incorporated into a variety of formulations and medicaments for therapeutic administration. More particularly, a compound as provided herein can be formulated into pharmaceutical compositions by combination with appropriate, pharmaceutically acceptable carriers, excipients and/or diluents, and can be formulated into preparations in solid, semi-solid, liquid or gaseous forms, such as tablets, capsules, pills, powders, granules, dragees, gels, slurries, ointments, solutions, suppositories, injections, inhalants and aerosols. As such, administration of the compounds can be achieved in various ways, including oral, buccal, rectal, parenteral, intraperitoneal, intradermal, transdermal, intracranial and/or intratracheal administration. Moreover, the compound can be administered in a local rather than systemic manner, in a depot or sustained release formulation. The compounds can be formulated with common excipients, diluents or carriers, and compressed into tablets, or formulated as elixirs or solutions for convenient oral administration, or administered by the intramuscular or intravenous routes. The compounds can be administered transdermally, and can be formulated as sustained release dosage forms and the like. The compounds can be administered alone, in combination with each other, or they can be used in combination with other known compounds.

Suitable formulations for use in the present invention are found in Remington's Pharmaceutical Sciences (Mack Publishing Company (1985) Philadelphia, PA, 17th ed.). Moreover, for a brief review of methods for drug delivery, see, Langer, Science (1990) 249:1527-1533.

Sustained-release preparations may be prepared. Suitable examples of sustained-release preparations include semi permeable matrices of solid hydrophobic polymers containing the compound of the invention, which matrices are in the form of shaped articles, e.g. films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and [gamma] ethyl-L-glutamate, non-degradable ethylene-vinyl acetate, degradable lactic acid-glycolic acid copolymers such as the LUPRON DEPOT(TM) (injectable microspheres composed of lactic acid-glycolic acid copolymer and leuprolide acetate), and poly-D-(-)-3-hydroxybutyric acid.

The compound of the present invention may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The pharmaceutical compositions described herein can be manufactured in a manner that is known to those of skill in the art, i.e., by means of conventional mixing, dissolving, granulating, dragee-making, levigating, emulsifying, encapsulating, entrapping or lyophilizing processes. The following methods and excipients are merely exemplary and are in no way limiting. For injection, the compound (and optionally another active agent) can be formulated into preparations by dissolving, suspending or emulsifying them in an aqueous or nonaqueous solvent, such as vegetable or other similar oils, synthetic aliphatic acid glycerides, esters of higher aliphatic acids or propylene glycol; and if desired, with conventional additives such as solubilizers, isotonic agents, suspending agents, emulsifying agents, stabilizers and preservatives. Preferably, the compounds of the present invention can be formulated in aqueous solutions, preferably in physiologically compatible buffers such as Hanks' solution, Ringer's solution, or physiological saline buffer. For transmucosal administration, penetrants appropriate to the barrier to be permeated are used in the formulation. Such penetrants are generally known in the art.

Preferably, pharmaceutical formulations for parenteral administration include aqueous solutions of the active compounds in water-soluble form. Additionally, suspensions of the active compounds can be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions can contain substances that increase the viscosity of the suspension, such as sodium carboxymethyl cellulose, sorbitol, or dextran. Optionally, the suspension can also contain suitable stabilizers or agents that increase the solubility of the compounds to allow for the preparation of highly concentrated solutions. Alternatively, the active ingredient can be in powder form for constitution with a suitable vehicle, e.g., sterile pyrogen-free water, before use.

The amount of a virucidal compound of the invention that can be combined with a carrier material to produce a single dosage form will vary depending upon the viral disease treated, the mammalian species, and the particular mode of administration. It will be also understood, that the specific dose level for any particular patient will depend on a variety of factors including the activity of the specific compound employed; the age, body weight, general health, sex and diet of the individual being treated; the time and route of administration; the rate of excretion; other drugs that have previously been administered; and the severity of the particular viral disease undergoing therapy, as is well understood by those of skill in the area.

Another aspect of the invention provides the virucidal compound of the invention for use in treating and/or preventing viral infections and viral diseases.

In some embodiments, the viruses are HSPG binding viruses. In other embodiments, the viruses are selected from, the group consisting of herpes simplex virus (HSV), human papillomavirus virus (HPV), respiratory syncytial virus (RSV), dengue virus and lentivirus (a human immunodeficiency virus (HIV) derived virus).

As used herein the terms "subject" or "patient" are well-recognized in the art, and, are used interchangeably herein to refer to a mammal, including dog, cat, rat, mouse, monkey, cow, horse, goat, sheep, pig, camel, and, most preferably, a human. In some embodiments, the subject is a subject in need of treatment or a subject beining infected by a virus, such as HSPG binding viruses. However, in other embodiments, the subject can be a healthy subject or a subject who has already undergone a treatment. The term does not denote a particular age or sex. Thus, adult, children and newborn subjects, whether male or female, are intended to be covered.

"Treatment" refers to both therapeutic treatment and prophylactic or preventative measures. Those in need of treatment include those already being infected by a virus, such as HSPG binding virus, as well as those in which the viral infection is to be prevented. Hence, the mammal, preferably human, to be treated herein may have been diagnosed as being infected by a virus, such as HSPG binding virus, or may be predisposed or susceptible to be infected by a virus, such as HSPG binding virus. Treatment includes ameliorating at least one symptom of, curing and/or preventing the development of a disease or condition due to viral infection. Preventing is meant attenuating or reducing the ability of a virus to cause infection or disease, for example by affecting a post-entry viral event.

"Mammal" for purposes of treatment refers to any animal classified as a mammal, including humans, domestic and farm animals or pet animals, such as dogs, horses, cats, cows, monkeys etc. Preferably, the mammal is human.

The term "therapeutically effective amount" refers to an amount of the compound of the invention effective to alter a virus, such as HSPG binding virus, and to render it inert, in a recipient subject, and/or if its presence results in a detectable change in the physiology of a recipient subjet, for example ameliorates at least one symptom of viral infection, prevents or reduces the rate transmission of at least one viral agent.

Another aspect of the invention provides a virucidal composition comprising an effective amount of the virucidal compound of the invention and optionally at least one suitable carrier. "An effective amount" refers to the amount sufficient for altering viruses, and/or destroying viruses and/or neutralizing viruses; i.e. sufficient for obtaining virucidal effect. In an embodiment, the suitable carrier is selected from the group comprising stabilisers, fragrance, colorants, emulsifiers, thickeners, wetting agents, or mixtures thereof. In another embodiment, the virucidal composition can be in the form of a liquid, a gel, a foam, a spray or an emulsion. In a further embodiment, the virucidal composition can be an air freshener, a sterilizing solution or a disinfecting solution.

Another aspect of the invention provides a device (or a product) comprising the virucidal composition of the invention and means for applying and/or dispensing the virucidal composition. In another embodiment, the means comprise a dispenser, a spray applicator or a solid support soaked with the virucidal composition. In another embodiment, the support is a woven or non-woven fabric, a textile, a paper towel, cotton wool, an absorbent polymer sheet, or a sponge.

Another aspect of the invention provides a method of disinfection and/or sterilization using the virucidal compounds of the invention or the virucidal composition of the invention.

In a preferred embodiment, the method of disinfection and/or sterilization comprises the steps of (i) providing at least one virucidal compound of the invention or the virucidal composition of the invention, (ii) contacting a virus contaminated surface or a surface suspected to be contaminated by viruses with the at least one virucidal compound of the invention or the virucidal composition of the invention for a time sufficient to obtain virucidal effet. Preferably the virus is HSPG binding virus; more preferably the virus is selected from the group consisting of herpes simplex virus (HSV), human papillomavirus virus (HPV), respiratory syncytial virus (RSV), dengue virus and lentivirus (a human immunodeficiency virus (HIV) derived virus). In some embodiments, the virus contaminated surface is human or animal skin. In other embodiments, the virus contaminated surface is a non-living surface, such as medical equipments, clothing, masks, furnitures, rooms, etc.

Another aspect of the invention provides a use of the virucidal compounds of the invention or the virucidal composition of the invention for sterilization and/or for disinfection. In some embodiments, sterilization and disinfection is for virus contamined surfaces or surfaces suspected to be contaminated by viruses. Preferably the virus is HSPG binding virus; more preferably virus is selected from the group consisting of herpes simplex virus (HSV), human papillomavirus virus (HPV), respiratory syncytial virus (RSV), dengue virus and lentivirus (a human immunodeficiency virus (HIV) derived virus). In some preferred embodiments, the surfaces are human or animal skin. In other preferred embodiments, the surfaces are non-living surfaces, such as medical equipments, clothing, masks, furnitures, rooms, etc. In an embodiment, the virucidal composition is used as virucidal hand disinfectant for frequent use. In another embodiment, the virucidal composition is applied by spraying. In a further embodiment, the virucidal composition is applied on a protective mask.

The foregoing description will be more fully understood with reference to the following Examples. Such Examples, are, however, exemplary of methods of practising the present invention and are not intended to limit the scope of the invention.

### Examples

### Methods

All starting materials were purchased from Sigma Aldrich and used as received unless stated otherwise. All aqueous solutions were made in deionized water treated with a Milli-Q^{™} reagent system ensuring a resistivity of ≥ 15 M**Ω**cm⁻¹.

### Synthesis of Heptakis-6-iodo-6-deoxy-beta-cyclodextrin (1): CD1

Triphenylphosphine (4g, 15.25 mmol, 22eq) in dry dimethyl formamide (DMF) (10 mL) was added iodine (4 g, 15.76 mmol, 22 eq) over a period of 10 min. This mixture was added to dried beta-cyclodextrin (800 mg) and the solution heated to 70°C for 8 hrs. Then the mixture was concentrated by half, cooled to 0°C and then sodium methoxide (3.35 g, 25 wt% solution in methanol) was added. The mixture was stirred for 1 hr at 0°C and then warmed to room temperature. Methanol (400 mL) was added to precipitate the product, which was collected. Solid was cleaned using soxhlet extraction with methanol overnight to remove excess iodine and triphenylphosphine and yield pure heptakis-6-iodo-6-deoxy-beta-cyclodextrin.

### Synthesis of CD2 (2)

**1** (CD1) (200 mg) was dissolved in dry dimethyl sulfoxide (DMSO) (10 mL) under nitrogen. 11-mercapto- 1-undecanesulfonate (MUS) (426.4 mg, 14 eq) was dissolved in dimethyl sulfoxide (10 mL) under nitrogen and mixed with the solution of **1**. Triethylamine (204.9 µL, 14 eq) was added and the mixture stirred at 60 °C for 3 days. The mixture was diluted with water and dialysed against water for 4 days at 60 °C (water changed twice per day). The solution was dried. If necessary precipitation (in diethyl ether) can be used.

### Inhibition Assays Lenti Virus (LV-VSV-G)

VSV-G pseudotyped lentivirus (LV-VSV-G), carrying GFP as reporter gene, was resuspended in PBS and incubated with increasing concentrations of commercial or as synthesized cyclodextrins in PBS for 1h at 37°C prior to cell infection. The mixture of virus/cyclodextrins was subsequently added to HeLa cells for LV-VSV-G. Transduction was stopped after 48h and cells were fixed with 1% p-formaldehyde (PFA) for 10-15 minutes at room temperature and resuspended in PBS. Transduction efficiency, calculated as the % GFP+ cells, of LV-VSV-G was measured by flow cytometry.

### Inhibition Assay HSV-2

The effect of cyclodextrins (CDs) on HSV infection was evaluated by a plaque reduction assay. Vero cells were pre-plated 24 h in advance in 24-well plates at a density of 10⁵ cells. Increasing concentrations of cyclodextrins were incubated with HSV-2 (multiplicity of infection (MOI) 0.0003 plaque forming units (pfu)/cell) at 37 °C for 1 hour and then the mixtures were added to the cells. Following virus adsorption (2 h at 37°C), the virus inoculum was removed, the cells were washed with medium and then overlaid with a medium containing 1.2 % methylcellulose. After 24 h (HSV-2) of incubation at 37°C, cells were fixed and stained with 0.1 % of crystal violet in 20 % ethanol and viral plaques were counted. The concentration producing 50 % reduction in plaque formation (IC₅₀) was determined using the Prism software by comparing drug-treated and untreated wells.

### Inhibition Assay HPV-16 PsV

293TT cells were preplated 24 h in advance in 96-well tissue culture-treated flat bottom plates at a density of 2×10⁴ cells/well in 100 µL of neutralization buffer (DMEM without phenol red, 10 % FBS, 1 % glutamate, 1 % nonessential amino acids, 1 % penicillin-streptomycin-fungizone, and 10 mM HEPES). Diluted PsV stocks (80 µL/well) were placed on 96-well non treated sterile, polystyrene plates (Nalge-Nunc, Roskilde, Denmark), combined with 20 µL of serially diluted cyclodextrins, and placed for 1 h at 37°C. The 100-µL PsV-compound mixture was transferred onto the pre-plated cells and incubated for 72 h. The final concentration of PsV was approximately 1 ng/mL L1. After incubation, 25 µL of supernatant was harvested. The SEAP content in the supernatant was determined using a Great Escape SEAP Chemiluminescence Kit (BD Clon-tech, Mountain View, CA) as directed by the manufacturer. 30 min after the addition of the substrate, samples were read using a Wallac 1420 Victor luminometer (PerkinElmer Life and Analytical Sciences, Inc., Wellesley, MA).

### Evaluation of virucidal activity against LV-VSV-G

The effect of NPs upon transduction on HeLa cells of recombinant LV-VSV-G (10⁶ TU/ml - MOI 10) was evaluated by incubating an effective inhibitory concentration of cyclodextrins (CDs) (100 µg/ml) with viruses for 2h at 37°C as previously described (Shogan, 2006). After incubation, transduction efficiency was determined by titration at high dilutions and calculated as the percentage of GFP+ cells by flow cytometry.

### Evaluation of virucidal activity against HSV-2, HPV-16

Viruses (10⁵ pfu for HSV-2 and HPV-16) and 100 µg/ml of cyclodextrins (CDs) were incubated at different time points (0, 5, 30, 60 or 120 min) at 37°C and then the virucidal effect was investigated with serial dilutions of the mixtures. Viral titers were calculated at dilutions at which the nanoparticle was not effective

### Viral Yield Reduction Assay

The assay is finalized to quantify the antiviral effect of compound testing its effect on the production of infectious viruses. Vero cells were seeded in 24-well plates at a density of 10⁵ cells/well and infected in duplicate with HSV-2 at a multiplicity of infection (MOI) of 0.01 plaque-forming units (pfu/cell) and in the presence of serial dilutions of the compound. Following adsorption at 37°C for 2 h, the virus inoculum was removed and cultures were grown in the presence of serial dilutions of cyclodextrins (CDs) until control cultures displayed extensive cytopathology. Supernatants were harvested and pooled as appropriate 24-48 h after infection and cell-free virus infectivity titers were determined in duplicate by plaque assay in Vero cell monolayers. The end-point of the assay was the effective concentration of cyclodextrins (CDs) that reduced virus yield by 50% (EC₅₀) compared to untreated virus controls.

### FACS analysis

Cells were trypsinized, washed with PBS and fixed in 1% PFA (paraformaldehyde) in PBS for 10 min at room temperature. Approximately 2×10⁴ events (cells) were analysed per sample and cells with no virus were used as negative control in order to determine the background of autofluorescence. Transduced cells were calculated as the percentage of GFP+ cells over the total population of analysed cells. The expression of the GFP protein on cells transduced with LV-VSV-G was assessed through the BD FACSCalibur^{™} flow cytometer (BD Biosciences) and data were analysed with BD CELLQuest^{™} software (BD Biosciences).

### Data analysis

All results (see Table 1) are presented as the mean values from three independent experiments. The EC₅₀ values for inhibition curves were calculated by regression analysis using the program GraphPad Prism version 5.0 (GraphPad Software, San Diego, California, U.S.A.) to fit a variable slope-sigmoidal dose-response curve. The selectivity indexes SI were calculated dividing the CC₅₀ for the EC₅₀.

**Table 1: results of the virucidal assay for CD1 and CD2**

| | Dilutions | | | |
|---|---|---|---|---|
| Sample | Stock | 1:10 | 1:100 | 1:1000 |
| Lentivirus | 51.79 | 7.61 | 1.12 | 0.50 |
| Lentivirus + CD1 0.5mg/mL | 85.49 | 27.38 | 6.26 | 1.31 |
| Lentivirus + CD2 0.0001mg/mL | 52.12 | 8.67 | 1.34 | 0.51 |
| Lentivirus + CD2 0.001mg/mL | 45.31 | 6.92 | 1.09 | 0.46 |
| Lentivirus + CD2 0.01mg/mL | 0.45 | 0.32 | 0.41 | 0.38 |
| Lentivirus + CD2 0.1mg/mL | 0.42 | 0.45 | 0.38 | 0.34 |
| Lentivirus + CD2 0.5mg/mL | 0.50 | 0.46 | 0.31 | 0.34 |

## Claims

1. A virucidal compound having formula (I) 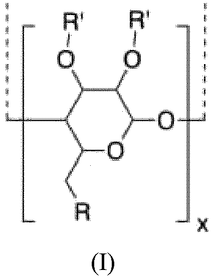 wherein:
x is 6, 7 or 8,
R is -Z-CH₂-(CH₂)_{y}-SO₃⁻,
Z is O or S,
y is at least 11,
y is at a maximum 25, and
R' is selected from the group consisting of H, -(CH₂)y-SO₃⁻ and -(CH₂)-COOH, or R' is a polymer selected from the group consisting of poly(ethylene glycol) (PEG), poly(vinyl alcohol) (PVA), poly(acrylamide) (PAAm), poly(n-butyl acrylate), poly-(**α**-esters), (PEG-b-PPO-b-PEG), poly(N-isopropylacrylamide) (pNIPAAM), polylacticglycolic acid (PLGA), dextran, dextrins, glucose, cellulose and cyclodextrins.

2. The virucidal compound of claim 1, wherein y is at maximum 20

3. The virucidal compound of any one of claims 1-2, wherein R' is H.

4. A virucidal compound comprising an alpha-cyclodextrin, a beta-cyclodextrin or a gamma-cyclodextrin functionalized on its primary face with at least six alkyl sulfate groups that provide an attachment receptor for HSPG binding viruses,
wherein said alkyl sulfate groups are at least C₇ groups, and
wherein said alkyl sulfate groups are at a maximum C₂₀ groups.

5. A pharmaceutical composition comprising an effective amount of one or more virucidal compounds of any one of claims 1-4 and at least one pharmaceutically acceptable excipient, carrier and/or diluent.

6. The virucidal compound for use in treating and/or preventing viral infections and viral diseases, wherein said virucidal compound is of any one of claims 1- 4 or of formula (I) 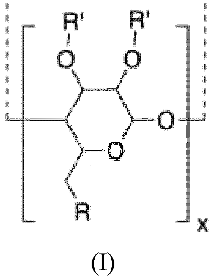 wherein:
x is 6, 7 or 8,
R is -Z-CH₂-(CH₂)_{y}-SO₃⁻,
Z is O or S,
y is at least 4, and
R' is selected from the group consisting of H, -(CH₂)y-SO₃⁻,- and -(CH₂)-COOH, or R' is a polymer selected from the group consisting of poly(ethylene glycol) (PEG), poly(vinyl alcohol) (PVA), poly(acrylamide) (PAAm), poly(n-butyl acrylate), poly-(**α**-esters), (PEG-b-PPO-b-PEG), poly(N-isopropylacrylamide) (pNIPAAM), polylacticglycolic acid (PLGA), dextran, dextrins, glucose, cellulose and cyclodextrins.

7. The virucidal compound for use in treating and/or preventing viral infections and viral diseases of claim 6, wherein the viruses are HSPG binding viruses.

8. The virucidal compound for use in treating and/or preventing viral infections and viral diseases of claim 7, wherein the viruses are selected from the group consisting of herpes simplex virus (HSV), human papillomavirus virus (HPV), respiratory syncytial virus (RSV), dengue virus and lentivirus (a human immunodeficiency virus (HIV) derived virus).

9. A virucidal composition comprising an effective amount of the virucidal compound of any one of claims 1-4 and optionally at least one suitable carrier.

10. A method of disinfection and/or sterilization comprising the steps of (i) providing at least one virucidal compound, (ii) contacting a virus contaminated surface or a surface suspected to be contaminated by viruses with the at least one virucidal compound for a time sufficient to obtain virucidal effect, wherein the virus contaminated surface is a non-living surface and wherein said virucidal compound is of any one of claims 1-4 or of formula (I) 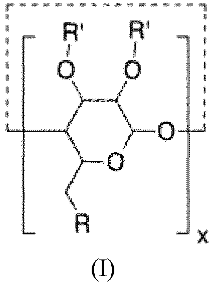 wherein:
x is 6, 7 or 8,
R is -Z-CH₂-(CH₂)_{y}-SO₃⁻,
Z is O or S,
y is at least 4, and
R' is selected from the group consisting of H, -(CH₂)y-SO₃⁻,- and -(CH₂)-COOH, or R' is a polymer selected from the group consisting of poly(ethylene glycol) (PEG), poly(vinyl alcohol) (PVA), poly(acrylamide) (PAAm), poly(n-butyl acrylate), poly-(**α**-esters), (PEG-b-PPO-b-PEG), poly(N-isopropylacrylamide) (pNIPAAM), polylacticglycolic acid (PLGA), dextran, dextrins, glucose, cellulose and cyclodextrins.

11. A device comprising the virucidal composition of claim 9 and means for applying and/or dispensing the virucidal composition.

12. A use of the virucidal compound for sterilization and/or for disinfection of virus contaminated surfaces or surfaces suspected to be contaminated by viruses, wherein the surfaces are non-living surfaces and wherein said virucidal compound is of any one of claims 1-4 or of formula (I) 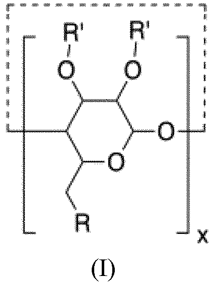 wherein:
x is 6, 7 or 8,
R is -Z-CH₂-(CH₂)_{y}-SO₃⁻,
Z is O or S,
y is at least 4, and
R' is selected from the group consisting of H, -(CH₂)_{y}-SO₃⁻,- and -(CH₂)-COOH, or R' is a polymer selected from the group consisting of poly(ethylene glycol) (PEG), poly(vinyl alcohol) (PVA), poly(acrylamide) (PAAm), poly(n-butyl acrylate), poly-(**α**-esters), (PEG-b-PPO-b-PEG), poly(N-isopropylacrylamide) (pNIPAAM), polylacticglycolic acid (PLGA), dextran, dextrins, glucose, cellulose and cyclodextrins.

## Patentansprüche

1. Viruzide Verbindung, mit der Formel (I) 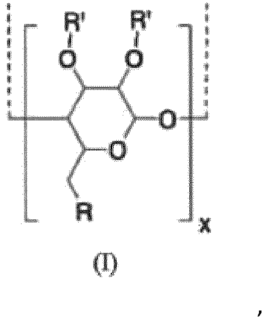 wobei:
x 6, 7 oder 8 ist,
R -Z-CH₂-(CH₂)_{y}-SO₃⁻ ist,
Z O oder S ist,
y mindestens 11 ist,
y maximal 25 ist, und
R' ausgewählt ist aus der Gruppe bestehend aus H, -(CH₂)_{y}-SO₃⁻ und -(CH₂)-COOH oder R' ein Polymer ist, ausgewählt aus der Gruppe bestehend aus Poly(ethylenglycol) (PEG), Poly(vinylalkohol) (PVA), Poly(acrylamid) (PAAm), Poly(n-butylacrylat), Poly-(α-ester), (PEG-b-PPO-b-PEG), Poly(N-isopropylacrylamid) (pNIPAAM), Poly(milch-co-glycol)säure (PLGA), Dextran, Dextrinen, Glucose, Cellulose und Cyclodextrinen.

2. Viruzide Verbindung nach Anspruch 1, wobei y maximal 20 ist.

3. Viruzide Verbindung nach einem der Ansprüche 1 bis 2, wobei R' H ist.

4. Viruzide Verbindung, die ein Alpha-Cyclodextrin, ein Beta-Cyclodextrin oder ein Gamma-Cyclodextrin umfasst, das auf seiner primären Seite mit mindestens sechs Alkylsulfatgruppen funktionalisiert ist, die einen Anheftungsrezeptor für HSPG-bindende Viren bereitstellen,
wobei die Alkylsulfatgruppen mindestens C₇-Gruppen sind, und
wobei die Alkylsulfatgruppen maximal C₂₀-Gruppen sind.

5. Arzneimittel, umfassend eine wirksame Menge einer oder mehrerer viruzider Verbindungen nach einem der Ansprüche 1 bis 4 und mindestens einen pharmazeutisch verträglichen Exzipienten, Träger und/oder mindestens ein pharmazeutisch verträgliches Verdünnungsmittel.

6. Viruzide Verbindung zur Verwendung bei der Behandlung und/oder Vorbeugung von viralen Infektionen und viralen Erkrankungen, wobei die viruzide Verbindung nach einem der Ansprüche 1 bis 4 oder der Formel (I) ist 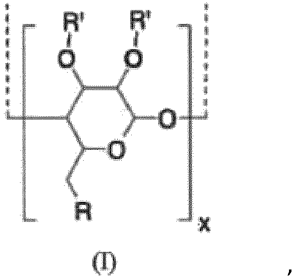 wobei:
x 6, 7 oder 8 ist,
R -Z-CH₂-(CH₂)_{y}-SO₃⁻ ist,
Z O oder S ist,
y mindestens 4 ist, und
R' ausgewählt ist aus der Gruppe bestehend aus H, -(CH₂)_{y}-SO₃⁻ und -(CH₂)-COOH oder R' ein Polymer ist, ausgewählt aus der Gruppe bestehend aus Poly(ethylenglycol) (PEG), Poly(vinylalkohol) (PVA), Poly(acrylamid) (PAAm), Poly(n-butylacrylat), Poly-(α-ester), (PEG-b-PPO-b-PEG), Poly(N-isopropylacrylamid) (pNIPAAM), Poly(milch-co-glycol)säure (PLGA), Dextran, Dextrinen, Glucose, Cellulose und Cyclodextrinen.

7. Viruzide Verbindung zur Verwendung bei der Behandlung und/oder Vorbeugung von viralen Infektionen und viralen Erkrankungen nach Anspruch 6, wobei die Viren HSPG-bindende Viren sind.

8. Viruzide Verbindung zur Verwendung bei der Behandlung und/oder Vorbeugung von viralen Infektionen und viralen Erkrankungen nach Anspruch 7, wobei die Viren ausgewählt sind aus der Gruppe bestehend aus Herpes-simplex-Virus (HSV), humanem Papillomavirus-Virus (HPV), respiratorischem Synzytial-Virus (RSV), Dengue-Virus und Lentivirus (einem von humanem Immundefizienz-Virus (HIV) abgeleiteten Virus).

9. Viruzide Zusammensetzung, umfassend eine wirksame Menge der viruziden Verbindung nach einem der Ansprüche 1 bis 4 und gegebenenfalls mindestens einen geeigneten Träger.

10. Verfahren zur Desinfektion und/oder Sterilisation, umfassend die Schritte des (i) Bereitstellens mindestens einer viruziden Verbindung, (ii) des Inkontaktbringens einer mit Viren kontaminierten Oberfläche oder einer Oberfläche, die im Verdacht steht, mit Viren kontaminiert zu sein, mit der mindestens einen viruziden Verbindung für eine Zeit, die ausreicht, um eine viruzide Wirkung zu erzielen, wobei die mit Viren kontaminierte Oberfläche eine nicht-lebende Oberfläche ist und wobei die viruzide Verbindung eine nach den Ansprüchen 1 bis 4 oder der Formel (I) ist 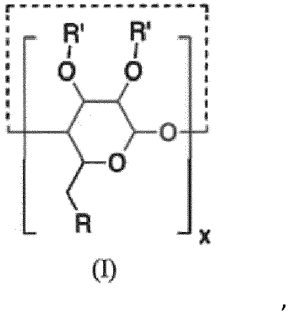 wobei:
x 6, 7 oder 8 ist,
R -Z-CH₂-(CH₂)_{y}-SO₃⁻ ist,
Z O oder S ist,
y mindestens 4 ist, und
R' ausgewählt ist aus der Gruppe bestehend aus H, -(CH₂)_{y}-SO₃⁻ und -(CH₂)-COOH oder R' ein Polymer ist, ausgewählt aus der Gruppe bestehend aus Poly(ethylenglycol) (PEG), Poly(vinylalkohol) (PVA), Poly(acrylamid) (PAAm), Poly(n-butylacrylat), Poly-(α-ester), (PEG-b-PPO-b-PEG), Poly(N-isopropylacrylamid) (pNIPAAM), Poly(milch-co-glycol)säure (PLGA), Dextran, Dextrinen, Glucose, Cellulose und Cyclodextrinen.

11. Vorrichtung, umfassend die viruzide Verbindung nach Anspruch 9 und Mittel zum Anwenden und/oder Dispensieren der viruziden Verbindung.

12. Verwendung der viruziden Verbindung zur Sterilisation und/oder zur Desinfektion von mit Viren kontaminierten Oberflächen oder Oberflächen, die im Verdacht stehen, mit Viren kontaminiert zu sein, wobei die Oberflächen nicht-lebende Oberflächen sind und wobei die viruzide Verbindung eine nach den Ansprüchen 1 bis 4 oder der Formel (I) ist 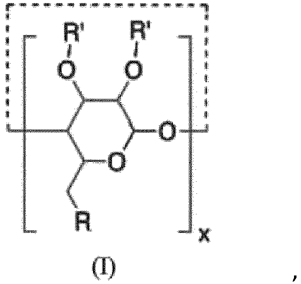 wobei:
x 6, 7 oder 8 ist,
R -Z-CH₂-(CH₂)_{y}-SO₃⁻ ist,
Z O oder S ist,
y mindestens 4 ist, und
R' ausgewählt ist aus der Gruppe bestehend aus H, -(CH₂)_{y}-SO₃⁻ und -(CH₂)-COOH oder R' ein Polymer ist, ausgewählt aus der Gruppe bestehend aus Poly(ethylenglycol) (PEG), Poly(vinylalkohol) (PVA), Poly(acrylamid) (PAAm), Poly(n-butylacrylat), Poly-(α-ester), (PEG-b-PPO-b-PEG), Poly(N-isopropylacrylamid) (pNIPAAM), Poly(milch-co-glycol)säure (PLGA), Dextran, Dextrinen, Glucose, Cellulose und Cyclodextrinen.

## Revendications

1. Composé virucide ayant la formule (I) 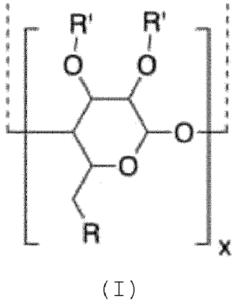 dans laquelle :
x est 6, 7 ou 8,
R est -Z-CH₂-(CH₂)_{y}-SO₃⁻,
Z est O ou S,
y est au moins 11,
y est au maximum de 25, et
R' est choisi dans le groupe constitué de
H, -(CH₂)_{y}-SO₃⁻ et -(CH₂)-COOH ou R'
est un polymère choisi dans le groupe constitué de : poly(éthylène glycol) (PEG), alcool polyvinylique (PVA), polyacrylamide (PAAm), poly(acrylate de n-butyle), poly-(a-esters), (PEG-b-PPO-b-PEG), poly(N-isopropylacrylamide) (pNIPAAM), poly(acide lactique-glycolique) (PLGA), dextrane, dextrines, glucose, cellulose et cyclodextrines.

2. Composé virucide selon la revendication 1, dans lequel y est au maximum de 20.

3. Composé virucide selon l'une quelconque des revendications 1 à 2, dans lequel R' est H.

4. Composé virucide comprenant une alpha-cyclodextrine, une bêta-cyclodextrine ou une gamma-cyclodextrine fonctionnalisée sur sa face primaire avec au moins six groupes alkylsulfate qui fournissent un récepteur de liaison pour des virus se liant à HSPG, dans lequel lesdits groupes alkylsulfate sont des groupes au moins en C₇, et
dans lequel lesdits groupes alkylsulfate sont des groupes au plus en C₂₀.

5. Composition pharmaceutique comprenant une quantité efficace d'un ou plusieurs composé virucides selon l'une quelconque des revendications 1 à 4 et au moins un excipient, véhicule et/ou diluant pharmaceutiquement acceptable.

6. Composé virucide pour utilisation dans le traitement et/ou la prévention d'infections virales et de maladies virales, ledit composé virucide étant selon l'une quelconque des revendications 1 à 4 ou de formule (I) 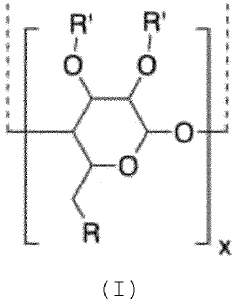 dans laquelle :
x est 6, 7 ou 8,
R est -Z-CH₂-(CH₂)_{y}-SO₃⁻,
Z est O ou S,
y est au moins 4, et
R' est choisi dans le groupe constitué de H, -(CH₂)_{y}-SO₃⁻ et -(CH₂)-COOH, ou R' est un polymère choisi dans le groupe constitué de : poly(éthylène glycol) (PEG), alcool polyvinylique (PVA), polyacrylamide (PAAm), poly(acrylate de n-butyle), poly-(a-esters), (PEG-b-PPO-b-PEG), poly(N-isopropylacrylamide) (pNIPAAM), poly(acide lactique-glycolique) (PLGA), dextrane, dextrines, glucose, cellulose et cyclodextrines.

7. Composé virucide pour utilisation dans le traitement et/ou la prévention d'infections virales et de maladies virales selon la revendication 6, les virus étant des virus se liant à HSPG.

8. Composé virucide pour utilisation dans le traitement et/ou la prévention d'infections virales et de maladies virales selon la revendication 7, les virus étant choisis dans le groupe constitué du virus de l'herpès simplex (HSV), le virus du papillome humain (VPH), le virus respiratoire syncytial (VRS), le virus de la dengue et un lentivirus (un virus dérivé du virus de l'immunodéficience humaine (VIH)).

9. Composition virucide comprenant une quantité efficace du composé virucide selon l'une quelconque des revendications 1 à 4 et facultativement au moins un véhicule adapté.

10. Procédé de désinfection et/ou stérilisation comprenant les étapes de (i) fourniture d'au moins un composé virucide, (ii) mise en contact d'une surface contaminée par un virus ou une surface suspectée d'être contaminée par des virus avec l'au moins un composé virucide pendant un temps suffisant pour obtenir un effet virucide, dans lequel la surface contaminée par un virus est une surface non vivante et dans lequel ledit composé virucide est selon l'une quelconque des revendications 1 à 4 ou de formule (I) 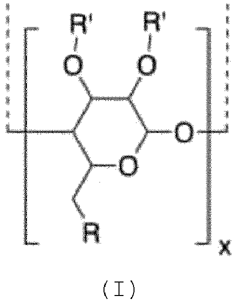 dans laquelle :
x est 6, 7 ou 8,
R est -Z-CH₂-(CH₂)_{y}-SO₃⁻,
Z est O ou S,
y est au moins 4, et
R' est choisi dans le groupe constitué de
H, -(CH₂)_{y}-SO₃⁻ et -(CH₂)-COOH, ou R'
est un polymère choisi dans le groupe constitué de : poly(éthylène glycol) (PEG), alcool polyvinylique (PVA), polyacrylamide (PAAm), poly(acrylate de n-butyle), poly-(a-esters), (PEG-b-PPO-b-PEG), poly(N-isopropylacrylamide) (pNIPAAM), poly(acide lactique-glycolique) (PLGA), dextrane, dextrines, glucose, cellulose et cyclodextrines.

11. Dispositif comprenant la composition virucide selon la revendication 9 et un moyen pour appliquer et/ou distribuer la composition virucide.

12. Utilisation du composé virucide pour la stérilisation et/ou pour la désinfection de surfaces contaminées par un virus ou de surfaces suspectées d'être contaminées par des virus, dans laquelle les surfaces sont des surfaces non vivantes et dans laquelle ledit composé virucide est selon l'une quelconque des revendications 1 à 4 ou de formule (I) 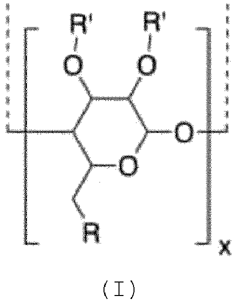 dans laquelle :
x est 6, 7 ou 8,
R est -Z-CH₂-(CH₂)_{y}-SO₃⁻,
Z est O ou S,
y est au moins 4, et
R' est choisi dans le groupe constitué de
H, -(CH₂)_{y}-SO₃⁻ et -(CH₂)-COOH, ou R'
est un polymère choisi dans le groupe constitué de : poly(éthylène glycol) (PEG), alcool polyvinylique (PVA), polyacrylamide (PAAm), poly(acrylate de n-butyle), poly-(a-esters), (PEG-b-PPO-b-PEG), poly(N-isopropylacrylamide) (pNIPAAM), poly(acide lactique-glycolique) (PLGA), dextrane, dextrines, glucose, cellulose et cyclodextrines.
